# EUROPEAN PATENT APPLICATION

(11) **EP 2 636 377 A1**
(43) Date of publication of application: **11.09.2013**
(21) Application number: 13382073.8
(22) Date of filing: 05.03.2013
(51) Int. Cl.: A61B 17/072

(54) **Surgical device**

(30) Priority: 05.03.2012 ES 201230330
(71) Applicant: Arteaga Gonzalez, Ivan Jesus, 38004 Santa Cruz De Tenerife (ES)
(72) Inventor: Arteaga Gonzalez, Ivan Jesus, 38004 Santa Cruz De Tenerife (ES)
(74) Representative: Carvajal y Urquijo, Isabel

(57) **Abstract**

Surgical device, suitable for coupling to the sleeve (5) of an endostaple with a means of actuation for a tightening channel (1) and a tightening anvil (2), wherein the tightening channel (1) includes a cartridge (3) with a knife (4) and with at least one line of pushers (8) for the staples on each side of the knife (4), and wherein the anvil (2) comprises a slot (6) for the knife (4) and at least one line of alveoli (7) on each side of said knife (4), said lines of alveoli (7) corresponding with the lines of pushers (8) for the staples, where the number of lines of pushers (8) and the number of lines of alveoli (7) for the staples is different on each side of the knife (4) and the slot (6) respectively.

## Description

### Object of the invention

The present invention relates to a surgical device for an endostapler that provides more safety in the resection edge that remains in the patient, and eliminating unnecessary staples in the edge to be removed. For this, the surgical device of the invention comprises a tightening channel and a tightening anvil of asymmetric or lateral localisation, in a way that there is a greater number of lines of pushers of staples and alveoli at one side of the knife and slot respectively, than on the opposite side.

### Background of the invention

In the state of the art, there is a plurality of post-operation stapling and tissue sectioning devices, commonly called surgical staplers or endostaplers.

A surgical stapler is a medical device that is used for placing surgical staples in the processes or operations that require resection of viscera.

Surgical staples have been developed over the years to provide more safety and rapidity in the resection of viscera, as well as in the performing of anastomosis, improving the results of traditional manual stitches, as the post-operation leaking and bleeding of the staple line are the most frequent causes of complications; including the death of patients.

The development of the surgical staplers provides advantages in speed of the operation, precision and uniformity in the performance of the procedures, making it an indispensable element in the operating theatre.

The current surgical endostaplers or staplers most used, have the double function of cutting and stapling. For performing these functions, they have a number of rows of staples arranged longitudinally which are placed at both sides of a central groove through which a knife is displaced which cuts the tissue at the same time as the ends of the cut are stapled. These instruments, in this way, facilitate surgical procedures in terms of rapidity, safety and uniformity, avoiding the technical errors of manual stitches.

The increase of lines of stitches or rows of staples, which has passed from four to six in the latest endostapler models, three lines on each side, increases the safety in the sealing of the tissues, reducing the number of incidents of bleeding or leaking.

However, linear endostaplers are especially designed for laparoscopic surgery and present a width limit of twelve millimetres so that its insertion is possible through the entry ports, used in this surgical approach, of twelve millimetres of maximum thickness. It has a head where a symmetrical or equivalent number of rows of staples are aligned, on both sides of the channel through which the knife that cuts passes. In this way, once sectioned, the tissues are hermetically sealed at both sides of the cut in an equal way, with two or three lines of staples maximum according to the model of endostapler used. This mechanism is especially useful in intestinal reconstructive surgery, i.e. when a joining or anastomosis between two viscera is performed, because a water-tight joining is important at both sides of the cut. However, in resection surgery, such as vertical gastrectomy, performed for the treatment of Pathological Obesity, one of the sides of the cut will correspond to the edge of the dried gastric remains, which will be removed from the patient. Therefore, this it is not necessary for this edge of resection to be reinforced with two or three lines of staples, as it will be removed. While, the edge of resection of the stomach remains in the patient and which will be the area of potential complications, will only have three lines of staples maximum.

This is translated in that the current surgical staples or endostaples, when they are used for resection surgery, systematically waste half of the rows of staples arranged at one side of the cut, as a consequence that they are apparatuses, conceptually manufactured for performing intestinal anastomosis. On the other hand, this mechanism also works suitably for procedures which require the same safety on the two ends of the section, for example, in the haemostasis of a large calibre vessel. However, in surgical procedures which involve the removal of the complete or partial organ, such as stomach or lung, the arrangement of an equivalent and symmetrical number of lines of stitches on the two sides of the cut is incoherent, because it equally reinforces the edge of the resection that remains and that which is removed from the body of the patient.

### Description of the invention

The invention proposed resolves the previously mentioned problem in a completely satisfactory way. It is a surgical device for an endostapler, provided with a head with a laterally displaceable channel section, having a greater number of rows of staples on one side than on the other side of the cut.

In this way, the edge of the resection of the viscera that is cut and that remains in the patient has more staple lines, while there are less staple lines on the edge of the resection that is to be extracted from the patient and therefore does not need to be reinforced. For example, in an endostaple of six rows of stables and standard size of 12 millimetres, up to five lines of staples are achieved in the edge that remains in the patient, while the edge that is extracted would have only one line of staples. In this way, the safety of the cut is increased substantially, significantly reducing the incidents of rupture and bleeding of the staple line and thus post-operative death.

The proposed surgical stapler allows using a head with the same width, a greater number of staples on the end of the section concerned; providing greater safety, displacing the knife and the slot to one side of the longitudinal central line of the channel and of the tightening anvil.

Therefore, because of this asymmetrical configuration, the proposed invention, which allows a greater number of staple lines on one side of the cut, provides more safety, in addition to allowing the possibility of designing smaller instruments than the current ones.

### Description of the drawings

To complete the description and with the aim of aiding a better understanding of the features of the invention, according to one practical embodiment thereof, attached as an integral part of the description, is a set of design plans wherein, by way of non-limitative example, the following has been represented:

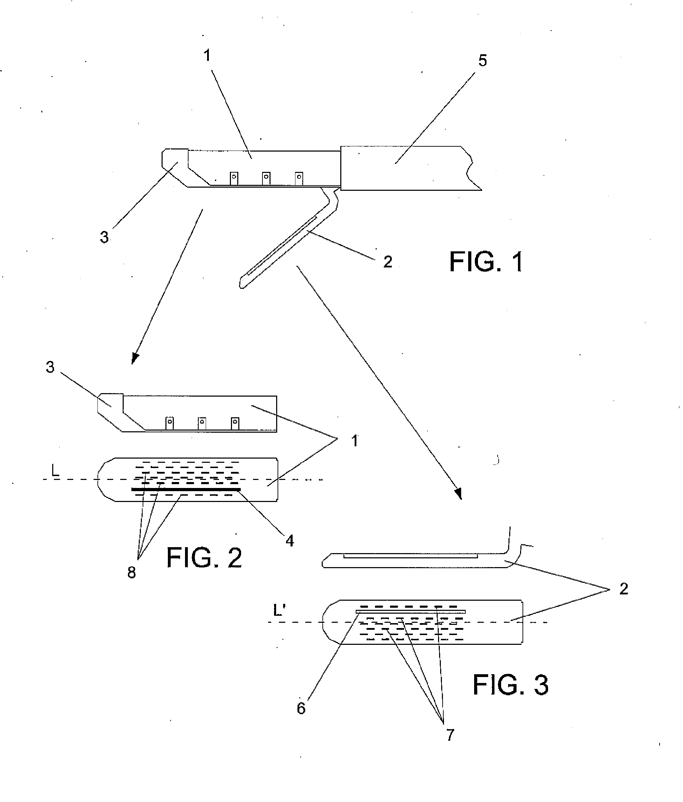

Figure 1.- shows a view of a possible preferred embodiment of the invention, being coupled to the sleeve of the endostapler.
Figure 2.- shows the tightening channel of the surgical device, in an elevational and plan view, the arrangement of the different staple lines being visible and wherein the knife is not in the centre of the tightening channel.
Figure 3.- shows the anvil of the surgical device of the invention, in elevational plan view, in which the plan, shows the arrangement of the alveoli for the staples and the slot for the knife.

### Preferred embodiment of the invention

The present invention describes a surgical device, suitable for being attached to the sleeve (5) of an endostapler comprising a means of actuation, which provides advantages with respect to the current devices.

The means of actuation of the endostapler acts on a tightening channel (1) and tightening anvil (2), wherein the tightening channel (1) includes a cartridge (3) with a knife (4) and with at least one line of pushers (8) for the staples on each side of the knife (4). The tightening anvil (2) comprises a slot (6) for the knife (4) and at least one line of alveoli (7) on each side of the knife (4). In this way, the number of lines of pushers (8) for the staples and lines of alveoli (7) for said staples is the same, the staples in the alveoli (7) coinciding in the tightening.

On order to achieve the object of the invention, where the resection edge that remains in the patient has a greater water-tightness and safety than the edge that is eliminated, the number of staples arranged on this edge of resection of the patient must be greater. For this, the number of lines of staple pushers (8), as well as the number of lines of alveoli (7) for the staples is different on each side of the knife (4) and the slot (6) respectively.

In this way, a device that has a greater number of staples on the resection side that remains in the patient is achieved, increasing safety and water-tightness.

Furthermore, it allows the design of smaller devices for the same number of rows of staple pushers (8), as it is displaced to one side of the knife (4) and the slot (6), enabling to increase the number of lines of staple pushers (8) of the desired side without the need to increase the size of the stapling device. In other words, both the knife (4) and the slot (6) are displaceable to one side of the longitudinal central line (L, L') of the tightening channel (1) and anvil (2).

Therefore, it is evident that it will be desired that the number of lines of pushers (8) for the staples and alveoli will be greater for the side of stapling of the edge of resection that remains inside the patient than for the side which is removed, saving costs in the disposed part and increasing water-tightness and safety in the edge of the resection of the patient.

## Claims

1. Surgical device, suitable for coupling to the sleeve (5) of an endostapler with a means of actuation for a tightening channel (1) and a tightening anvil (2), comprising a means of actuation for a tightening channel (1) and a tightening anvil (2), wherein the tightening channel (1) includes a cartridge (3) with a knife (4) and at least one line of pushers (8) for the staples on each side of the knife (4), and where the anvil (2) comprises a slot (6) for the knife (4) and a least one line of alveoli (7) on each side of said knife (4), said lines of alveoli (7) corresponding with the lines of pushers (8) for the staples, **characterised in that** the number of lines of pushers (8) and the number of lines of alveoli (7) for the staples is different on each side of the knife (4) and the slot (6) respectively.

2. Surgical device, according to claim 1, **characterised in that** both the knife (4) and the slot (6) are displaceable to one side of the longitudinal central line (L, L') of the tightening channel (1) and anvil (2).

3. Surgical device, according to previous claims, **characterised in that** the number of lines of pushers (8) for the staples and the number of lines of alveoli (7) is greater for the side of the stapling of the edge of the resection that remains in the patient.
